(19) Europäisches Patentamt
European Patent Office
Office européen des brevets

(11) Veröffentlichungsnummer : **0 057 846**
**B1**

(12) # EUROPÄISCHE PATENTSCHRIFT

(45) Veröffentlichungstag der Patentschrift : **05.06.85**

(51) Int. Cl.⁴ : **C 07 D207/273**, A 61 K 31/40

(21) Anmeldenummer : **82100494.2**

(22) Anmeldetag : **25.01.82**

(54) **Pyrrolidin-Derivat, Verfahren und Zwischenprodukte zu dessen Herstellung und dieses enthaltende Arzneimittel.**

(30) Priorität : **05.02.81 CH 770/81**

(43) Veröffentlichungstag der Anmeldung : **18.08.82 Patentblatt 82/33**

(45) Bekanntmachung des Hinweises auf die Patenterteilung : **05.06.85 Patentblatt 85/23**

(84) Benannte Vertragsstaaten : **AT BE CH DE FR IT LI LU NL SE**

(56) Entgegenhaltungen :
**EP-A- 0 005 143**
**EP-A- 0 024 030**
**Chemical Abstracts Band 87, Nr. 5 1. August 1977 Columbus, Ohio, USA S.A. SPAN "2-Pyrrolidinone derivative" Seite 500, Spalte 1, Abstract Nr. 39272m**

(73) Patentinhaber : **F. HOFFMANN-LA ROCHE & CO. Aktiengesellschaft**
**CH-4002 Basel (CH)**

(72) Erfinder : **Aschwanden, Werner**
**Grellingerstrasse 4**
**CH-4107 Ettingen (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Patentanwälte Dr. Franz Lederer Dipl.-Ing. Reiner F. Meyer-Roxlau Lucile-Grahn-Strasse 22**
**D-8000 München 80 (DE)**

**EP 0 057 846 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Pyrrolidin-Derivat. Im Speziellen betrifft sie das 1-(3-Hydroxy-4-methoxybenzoyl)-3-hydroxy-2-pyrrolidinon der Formel

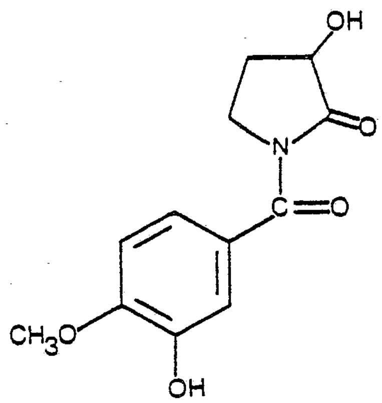

(I)

Diese Verbindung ist neu und zeichnet sich durch wertvolle pharmakodynamische Eigenschaften aus.

Gegenstand der vorliegenden Erdindung sind das 1-(3-Hydroxy-4-methoxybenzoyl)-2-pyrrolidinon der Formel I als solches und als pharmazeutischer Wirkstoff, die Herstellung dieser Verbindung und Zwischenprodukte für die Herstellung dieser Verbindung, ferner Arzneimittel, enthaltend die Verbindung der Formel I und die Herstellung solcher Arzneimittel, sowie die Verwendung der Verbindung der Formel I bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung der intellektuellen Leistungsfähigkeit.

Die Verbindung der Formel I enthält ein asymmetrisches Kohlenstoffatom ; die vorliegende Erfindung umfasst sowohl die optisch einheitlichen enantiomeren Formen dieser Verbindung als auch Gemische davon (insbesondere das Racemat).

Das Pyrrolidin-Derivat der Formel I kann erfindungsgemäss dadurch hergestellt werden, dass man aus einem Pyrrolidin-Derivat der allgemeinen Formel

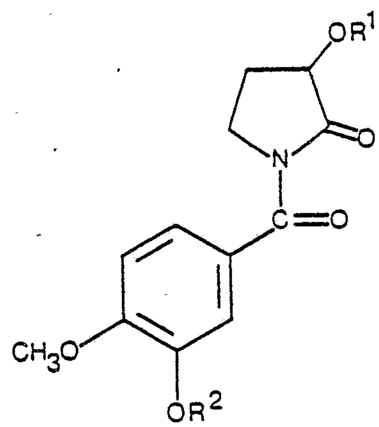

(II)

worin eines von $R^1$ und $R^2$ eine Schutzgruppe und das andere Wasserstoff oder eine Schutzgruppe bedeutet, die Schutzgruppe(n) entfernt.

Als Schutzgruppen in den Pyrrolidin-Derivaten der allgemeinen Formel II eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Die Entfernung der Schutzgruppe(n) aus den Pyrrolidin-Derivaten der allgemeinen Formel II erfolgt nach an sich bekannten Methoden, wobei natürlich für die Wahl der zur Anwendung gelangenden Methode die Natur der zu entfernenden Schutzgruppe(n) in Betracht gezogen werden muss und zu beachten ist, dass nur die Schutzgruppe(n) selektiv entfernt, andere im Molekül vorhandene Strukturelemente jedoch nicht in Mitleidenschaft gezogen werden sollen.

Die eingangs definierte Formel II umfasst erstens Verbindungen der allgemeinen Formel

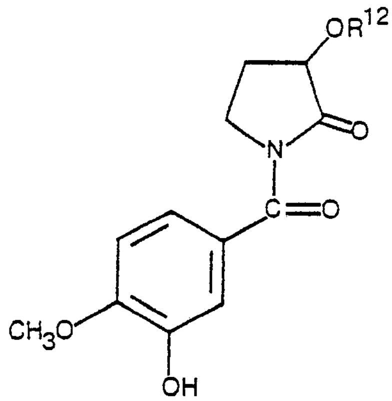

(IIa)

worin $R^{12}$ eine Schutzgruppe bedeutet, zweitens Verbindungen der Formel

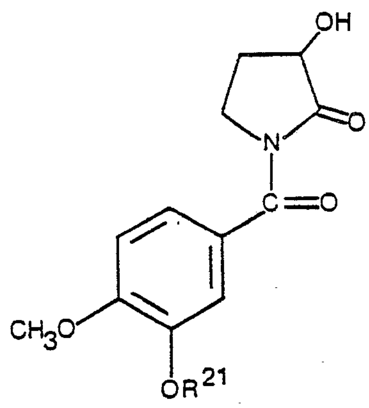

(IIb)

worin $R^{21}$ eine Schutzgruppe bedeutet, und drittens Verbindungen der Formel

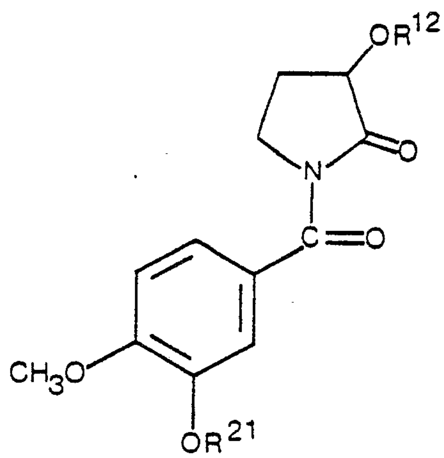

(IIc)

worin $R^{12}$ und $R^{21}$ je eine Schutzgruppe bedeuten.

Als Schutzgruppen, welche durch das Symbol $R^{12}$ wiedergegeben werden, eignen sich beispielsweise leicht abspaltbare Alkyl- und Aralkylgruppen, wie substituierte Tritylgruppen (z. B. p-Methoxytrityl, p,p'-Dimethoxytrityl oder p,p',p'''-Trimethoxytrityl) und dergleichen ; leicht abspaltbare metallorganische Gruppen, insbesondere Trialkylsilylgruppen, wie Trimethylsilyl, und dergleichen ; leicht abspaltbare Acetal- und Ketalschutzgruppen, wie Tetrahydropyran-2-yl, 4-Methoxytetrahydropyran-4-yl und dergleichen ; leicht abspaltbare Acylgruppen, wie Acetyl, Chloracetyl, Trifluoracetyl, Methoxyacetyl, Phenoxyacetyl, Benzyloxycarbonyl, Trichloräthoxyçarbonyl, Tribromäthoxycarbonyl, Benzoylformyl und dergleichen ; usw.

Methoden für die Entfernung der Reste, welche vorstehend als Beispiele für durch das Symbol $R^{12}$ bezeichnete Schutzgruppen erwähnt wurden, sind in der Literatur beschrieben und demnach jedem Fachmann geläufig. Beispielsweise kann man die erwähnten Mono-, Di- und Trimethoxytritylgruppen durch Behandeln mit 80 %iger Essigsäure bei Raumtemperatur abspalten, die Trimethylsilylgruppe durch Behandeln mit verdünnter Salzsäure in Tetrahydrofuran oder dergleichen, die Tetrahydropyran-2-yl-

gruppe und die 4-Methoxytetrahydropyran-4-ylgruppe unter milden sauren wässrigen Bedingungen (z. B. mittels 0,1 normaler Salzsäure), die Acetylgruppe mittels Esterase- Enzymen, die Chloracetylgruppe mittels Thioharnstoff/Pyridin, die Trifluoracetylgruppe mittels Methanol, die Methoxyacetyl- und die Phenoxyacetylgruppe mittels methanolischem Ammoniak, die Benzyloxycarbonylgruppe durch katalytische Hydrierung (z. B. über Palladium/Kohle), die Trichloräthoxycarbonyl- und die Tribromäthoxycarbonylgruppe mittels Zink-Kupfer in Eisessig bei Raumtemperatur und die Benzoylformylgruppe durch Behandeln mit wässrigem Pyridin bei Raumtemperatur.

Als Schutzgruppen, welche durch das Symbol $R^{21}$ bezeichnet werden, eignen sich beispielsweise leicht abspaltbare Alkylgruppen, wie tert.Butyl und dergleichen ; leicht abspaltbare Aralkylgruppen, wie Benzyl und dergleichen ; leicht abspaltbare metallorganische Gruppen, insbesondere Trialkylsilylgruppen, wie Trimethylsilyl und dergleichen ; leicht abspaltbare Acetal- und Ketalschutzgruppen wie Tetrahydropyran-2-yl und dergleichen ; leicht abspaltbare Acylgruppen wie Fluorencarbonyl, Benzyloxycarbonyl, Trichloräthoxycarbonyl, Tribromäthoxycarbonyl und dergleichen.

Methoden für die Abspaltung der Reste, welche vorstehend als Beispiele für durch das Symbol $R^{21}$ bezeichnete Schutzgruppen erwähnte wurden, sind in der Literatur beschrieben und demnach jedem Fachmann geläufig. So lassen sich beispielsweise die Benzyl- und die Benzyloxycarbonylgruppe durch katalytische Hydrierung entfernen (z. B. über Palladium/Kohle), die tert.Butyl-, die Trimethylsilyl- und die Tetrahydropyran-2-ylgruppe unter milden sauren wässrigen Bedingungen, die Fluorencarbonylgruppe mittels UV-Licht und die Trichloräthoxycarbonyl- und die Tribromäthoxycarbonylgruppe durch Erhitzen in Methanol oder mittels Zink-Kupfer in Eisessig.

Beispiele für Verbindungen der Formel II sind :

1-(3-Hydroxy-4-methoxybenzoyl)-2-oxo-3-pyrrolidinylacetat (A),
1-(3-Benzyloxy-4-methoxybenzoyl)-3-hydroxy-2-pyrrolidinon (B),
3-(Benzyloxycarbonyloxy)-1-(3-benzyloxy-4-methoxybenzoyl)-2-pyrrolidinon (C),
1-(3-Benzyloxy-4-methoxybenzoyl)-2-oxo-3-pyrrolidinyltrifluoracetat (D) und
1-(3-Benzyloxy-4-methoxybenzoyl)-2-oxo-3-pyrrolidinylacetat (E).

Bei der vorstehend als A bezeichneten Verbindung handelt es sich um eine Verbindung der Formel IIa, und zwar um diejenige, worin $R^{12}$ Acetyl bedeutet. Diese Verbindung kann mittels Esterase-Enzymen in die eingangs erwähnte Verbindung der Formel I übergeführt werden.

Bei der vorstehend als B bezeichneten Verbindung handelt es sich um eine Verbindung der Formel IIb, und zwar um diejenige, worin $R^{21}$ Benzyl bedeutet. Diese Verbindung kann durch katalytische Hydrierung (z. B. über Palladium/Kohle) in die eingangs erwähnte Verbindung der Formel I übergeführt werden.

Bei den vorstehend als C, D und E bezeichneten Verbindungen handelt es sich um Verbindungen der Formel IIc, und zwar im Falle von C um diejenige, worin $R^{12}$ Benzyloxycarbonyl und $R^{21}$ Benzyl bedeuten, im Falle von D um diejenige, worin $R^{12}$ Trifluoracetyl und $R^{21}$ Benzyl bedeuten, und im Falle von E um diejenige, worin $R^{12}$ Acetyl und $R^{21}$ Benzyl bedeuten. Verbindung C ist ein Beispiel für eine Verbindung der Formel IIc, welche in einem Arbeitsgang unter Abspaltung beider Schutzgruppen in die eingangs erwähnte Verbindung der Formel I übergeführt werden kann ; dies kann durch katalytische Hydrierung erfolgen, beispielsweise über Palladium/Kohle. Verbindung D ist ein Beispiel für eine Verbindung der Formel IIc, welche in zwei Arbeitsgängen in die eingangs erwähnte Verbindung der Formel I übergeführt werden kann ; beispielsweise kann man durch Abspaltung der Trifluoracetylgruppe mittels Methanol zunächst zu Verbindung B gelangen, welche dann, wie bereits erwähnt, durch katalytische Hydrierung (z. B. über Palladium/Kohle) in die eingangs erwähnte Verbindung der Formel I übergeführt werden kann. Die Verbindung E ist ein weiteres Beispiel für eine Verbindung der Formel IIc, welche in zwei Arbeitsgängen in die eingangs erwähnte Verbindung der Formel I übergeführt werden kann ; beispielsweise kann man durch Abspaltung der Benzylgruppe durch katalytische Hydrierung (z. B. über Palladium/Kohle) zu Verbindung A gelangen, welche dann, wie bereits erwähnt, mittels Esterase-Enzymen in die eingangs erwähnte Verbindung der Formel I übergeführt werden kann. Aufgrund der Natur der beiden Schutzgruppen und unter Berücksichtigung der für die Entfernung dieser Schutzgruppen zur Verfügung stehenden Methoden ist es dem Fachmann für jede Verbindung der Formel IIc klar, ob sie in einem Arbeitsgangs oder in zwei Arbeitsgängen in die eingangs erwähnte Verbindung der Formel I übergeführt werden kann.

Die Ausgangsprodukte der eingangs definierten allgemeinen Formel II sind neu und ebenfalls Gegenstand der vorliegenden Erfindung.

Verbindungen der Formel II, worin $R^1$ und $R^2$ je eine Schutzgruppe bedeuten, d. h. Verbindungen der allgemeinen Formel IIc, können beispielsweise dadurch hergestellt werden, dass man ein Pyrrolidin-Derivat der allgemeinen Formel

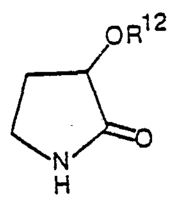

(III)

worin $R^{12}$ obige Bedeutung besitzt, in 1-Stellung entsprechend acyliert, d. h. das Wasserstoffatom in 1-Stellung der Verbindung der Formel III durch einen entsprechend substituierten Benzoylrest ersetzt. Hierbei kann man Methoden verwenden, welche für derartige Acylierungen an sich allgemein bekannt sind. Als Acylierungsmittel verwendet man ein hinreichend reaktionsfähiges Derivat einer Säure der allgemeinen Formel

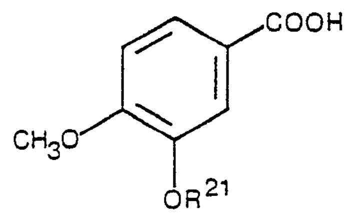

(IV)

worin $R^{21}$ obige Bedeutung besitzt, insbesondere ein reaktionsfähiges Imidazolid oder Halogenid einer solchen Säure (beispielsweise 3-Benzyloxy-4-methoxybenzoylchlorid).

Bei dieser Acylierung ist es zweckmässig, die Verbindung der Formel III zunächst mit einer zur Abstraktion des Wasserstoffatoms am Stickstoffatom in 1-Stellung befähigten Base zu behandeln (z. B. mit Butyllithium) und hierauf mit dem reaktionsfähigen Derivat der Säure der Formel IV reagieren zu lassen. Es ist auch möglich, die Verbindung der Formel III in Form eines reaktionsfähigen Derivates einzusetzen, in welchem am Stickstoffatom in 1-Stellung eine leicht abspaltbare Gruppe sitzt, insbesondere eine Trialkylsilylgruppe, wie 1-Trimethylsilyl ; in diesem Fall kommen natürlich als Schutzgruppen ($R^{12}$) nur Reste in Frage, welche unter den Bedingungen der Acylierung nicht in Mitleidenschaft gezogen werden.

Die Verbindungen der Formel III können ihrerseits beispielsweise aus 3-Hydroxy-2-pyrrolidinon hergestellt werden, und zwar durch Einführung der gewünschten Schutzgruppe ; die Methoden zur Einführung der Schutzgruppen variieren je nach deren Natur, sind jedoch jedem Fachmann geläufig. Beispielsweise kann man eine Benzyloxycarbonylgruppe mittels Chlorameisensäurebenzylester einführen.

Gewisse Verbindungen der allgemeinen Formel III können auch aus 4-Amino-2-hydroxybuttersäure hergestellt werden, und zwar durch Methoden, welche in einem Arbeitsgang Cyclisation und Einführung der gewünschten Schutzgruppe bewirken. So kann man beispielsweise zu 3-(Trimethylsilyloxy)-2-pyrrolidinon gelangen, indem man 4-Amino-2-hydroxybuttersäure in Gegenwart geringer Menge von Trimethylchlorsilan mit Hexamethyldisilazan oder mit bis-(Trimethylsilyl)harnstoff oder mit bis-(Trimethylsilyl)acetamid umsetzt.

Andererseits ist es auch möglich, Verbindungen der Formel IIc aus Verbindungen der allgemeinen Formel

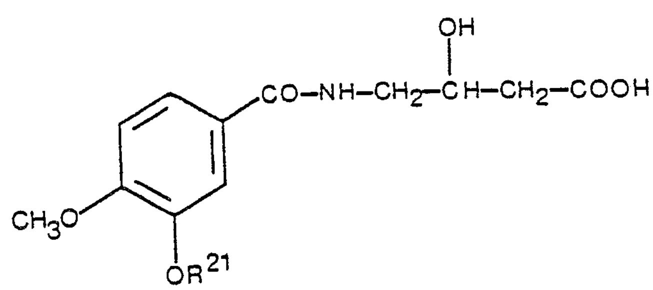

(V)

worin $R^{21}$ obige Bedeutung besitzt, herzustellen, welche ihrerseits durch Acylierung von 4-Amino-2-hydroxybuttersäure mit einem reaktionsfähigen Derivat einer Säure der Formel IV (beispielsweise mit 3-Benzyloxy-4-methoxybenzoylchlorid) zugänglich sind. So erfolgt beispielsweise bei der Behandlung von 4-[(3-Benzyloxy-4-methoxybenzoyl)-amino]-2-hydroxybuttersäure mit Essigsäureanhydrid in einem Arbeitsgang Cyclisation und Einführung der Schutzgruppe, d. h. man erhält eine Verbindung der Formel IIc, worin $R^{21}$ Benzyl und $R^{12}$ Acetyl bedeuten. Beispiele für weitere Reagenzien, mit welchen die 4-[(3-Benzyloxy-4-methoxybenzoyl)amino]-2-hydroxybuttersäure oder eine andere Verbindung der Formel V in einem Arbeitsgang in eine Verbindung der Formel IIc überführt werden kann, sind Chloressigsäureanhydrid, Methoxyessigsäureanhydrid, Trifluoressigsäureanhydrid, Hexamethyldisilazan und dergleichen ; in der erhaltenen Verbindung der Formel IIc bedeutet $R^{22}$, entsprechend dem verwendeten Reagens, Chloracetyl bzw. Methoxyacetyl bzw. Trifluoracetyl bzw. Trimethylsilyl oder dergleichen.

Weiterhin ist es auch möglich, Derivate von Verbindungen der Formel V, deren Hydroxygruppe geschützt ist, zu entsprechenden Verbindungen der Formel IIc zu cyclisieren ; zur Herstellung der erwähnten Derivate der Verbindungen der Formel V geht man von Derivaten der 4-Amino-2-hydroxybuttersäure aus, deren Hydroxygruppe bereits durch die gewünschte Schutzgruppe geschützt ist (und welche nach an sich bekannten Methoden leicht hergestellt werden können), und acyliert deren Aminogruppe mit einem hinreichend reaktiven Derivat einer Verbindung der Formel IV.

Zu Verbindungen der Formeln IIa und IIb kann man gelangen, indem man aus einer geeigneten Verbindung der Formel IIc eine der beiden Schutzgruppen entfernt. So kann man beispielsweise die weiter oben erwähnte Verbindung E (Formel IIc, worin $R^{12}$ Acetyl und $R^{21}$ Benzyl bedeuten) durch Hydrierung in Gegenwart von Palladium/Kohle in die weiter oben erwähnte Verbindung A überführen, d. h. in die Verbindung der Formel IIa, worin $R^{12}$ Acetyl bedeutet. Weiterhin kann man beispielsweise die weiter oben erwähnte Verbindung D (Formel IIc, worin $R^{12}$ Trifluoracetyl und $R^{21}$ Benzyl bedeuten) mittels Methanol in die weiter oben erwähnte Verbindung B überführen, d. h. in die Verbindung der Formel IIb, worin $R^{21}$ Benzyl bedeutet. Aufgrund der Natur der beiden Schutzgruppen und der für die Abspaltung dieser Schutzgruppen verfügbaren Methoden ist es dem Fachmann für jede Verbindung der Formel IIc klar, ob aus ihr eine der beiden Schutzgruppen selektiv entfernt werden kann, ohne dass die andere Schutzgruppe dabei in Mitleidenschaft gezogen wird und, bejahendenfalls, ob die fragliche Verbindung der Formel IIc in die entsprechende Verbindung der Formel IIa oder in die entsprechende Verbindung der Formel IIb übergeführt werden kann.

Die Verbindungen der allgemeinen Formel II weisen in 3-Stellung des 5-gliedrigen Heterocyclus ein asymmetrisches Kohlenstoffatom auf. Die diesbezüglichen stereochemischen Verhältnisse bestimmen die stereochemischen Verhältnisse bei der aus den Verbindungen der Formel II herstellbaren Verbindung der Formel I, d. h. 1-(3-Hydroxy-4-methoxybenzoyl)-3-hydroxy-2-pyrrolidinon. Die stereochemischen Verhältnisse in 3-Stellung des 5-gliedrigen Heterocyclus der Verbindungen der Formel II werden ihrerseits durch die bei der Herstellung der Verbindungen der Formel II verwendeten Vorprodukte und/oder Methoden bestimmt. Es ist für jeden Fachmann klar, wie er im Hinblick auf die soeben geschilderten Verhältnisse erfindungsgemäss zu optisch aktivem oder racemischem 1-(3-Hydroxy-4-methoxybenzoyl)-3-hydroxy-2-pyrrolidinon gelangen kann.

So kann man beispielsweise zu (R)-1-(3-Hydroxy-4-methoxybenzoyl)-3-hydroxy-2-pyrrolidinon gelangen, indem man (R)-4-Amino-2-hydroxybuttersäure mittels 3-Benzyloxy-4-methoxybenzoylchlorid acyliert, die erhaltene (R)-4-[(3-Benzyloxy-4-methoxybenzoyl)amino]-2-hydroxybuttersäure mittels Trifluoressigsäureanhydrid in (R)-1-(3-Benzyloxy-4-methoxybenzoyl)-2-oxo-3-pyrrolidinyl-trifluoracetat überführt, aus diesem die Trifluoracetylgruppe abspaltet und aus dem erhaltenen (R)-1-(3-Benzyloxy-4-methoxybenzoyl)-3-hydroxy-2-pyrrolidinon die Benzylgruppe abspaltet.

In analoger Weise kann man ausgehend von (S)-4-Amino-2-hydroxybuttersäure zu (S)-1-(3-Hydroxy-4-methoxybenzoyl)-3-hydroxy-2-pyrrolidinon und ausgehend von (R,S)-4-Amino-2-hydroxybuttersäure zu (R,S)-1-(3-Hydroxy-4-methoxybenzoyl)-3-hydroxy-2-pyrrolidinon gelangen.

Wie eingangs erwähnt, ist das Pyrroldin-Derivat der Formel I eine neue Verbindung mit äusserst wertvollen pharmakodynamischen Eigenschaften. Sie weist nur eine geringe Toxizität auf, und es hat sich gezeigt, dass sie in dem nachstehend beschriebenen Tierversuch experimentell hervorgerufener cerebraler Insuffizienz entgegenzuwirken vermag.

Die Test-Apparatur ist eine « Skinner box » mit einem elektrifizierbaren Gitterboden (30 × 40 cm) und einer grauen Plastik-Plattform (15 × 15 × 0,8 cm) in der Ecke vorne rechts. Unerfahrene männliche Ratten (100-120 g) werden einzeln auf die Plattform gebracht. Sobald sie auf den Gitterboden hinabsteigen, erhalten sie einen elektrischen Fuss-Schock (0,8 mA). Die normale Reaktion unerfahrener Ratten ist, daraufhin auf die Plattform zurückzuspringen. Da jedoch die Ratten immer nochmals herabzuklettern versuchen, muss die Fuss-Schock-Prozedur für jedes Tier drei- bis fünfmal wiederholt werden. Nach diesen drei bis fünf Wiederholungen pro Tier haben die Ratten eine sogenannte « passive avoidance response » erlernt, d. h. sie versuchen nicht mehr, auf den Gitterboden hinabzusteigen, weil sie wissen, dass sie bestraft werden.

Unmittelbar anschliessend werden drei Gruppen von je 30 Tieren gebildet. Die erste Gruppe erhält eine Injektion (i. p.) von 0,3 mg/kg Scopolamin sowie destilliertes Wasser (p. o./2 ml/kg). Die zweite Gruppe erhält eine Injektion (i. p.) von 0,3 mg/kg Scopolamin und eine orale Dosis der Testsubstanz. Die dritte Gruppe erhält ausschliesslich destilliertes Wasser (p. o.).

2 Stunden später wird jede Ratte einmal auf die Plattform in der «Skinner box » gesetzt. Das Kriterium für die Beurteilung dieses Tests zur Ermittlung einer Präparatwirkung auf das Kurzzeit-Gedächtnis ist, ob das Tier während 60 Sekunden auf der Plattform bleibt oder nicht (das Ergebnis kann also für jedes Tier nur « ja » oder « nein » lauten). Die statistische Signifikanz der Unterschiede zwischen den bei der ersten und der zweiten Gruppe erhaltenen Resultaten wird mittels des Chi-Quadrat-Tests ermittelt.

70-75 % der lediglich mit destilliertem Wasser (p. o.) behandelten Tiere erinnern sich 2-4 Stunden nach Erlernen der « passive avoidance response » noch daran, dass sie auf der Plattform bleiben sollten. Bei 85-92 % der mit Scopolamin (0,3 mg/kg i. p.) und destilliertem Wasser (p. o.) behandelten Tiere lässt sich während 3-4 Stunden ein retrograder Effekt auf das Kurzzeitgedächtnis feststellen, d. h. sie haben vergessen, dass sie auf der Plattform bleiben müssen. Eine Substanz, welche cerebraler Insuffizienz entgegenzuwirken vermag, kann die durch die Injektion (i. p.) von 0,3 mg/kg Scopolamin hervorgerufene Blockierung des Kurzzeitgedächtnisses aufheben. Eine Dosis eines Präparats wird dann als « aktiv » gegen Scopolamin bezeichnet, wenn die Anzahl der positiven Ergebnisse (« ja ») von derjenigen mit Scopolamin (0,3 mg/kg i. p.) und nur destilliertem Wasser (p. o.) behandelter Kontroll-Tiere signifikant verschieden ist.

In der nachfolgenden Tabelle ist angegeben, bei welchen Dosen das Racemat und die beiden optisch einheitlichen enantiomeren Formen der Verbindung der Formel I in dem vorstehend beschriebenen

Versuch eine signifikante Aktivität zeigen. Die Tabelle enthält ausserdem Angaben über die akute Toxizität ($DL_{50}$ in mg/kg bei einmaliger oraler Verabreichung an Mäuse).

| Konfiguration | Signifikant wirksame Dosen | DL 50 |
|---|---|---|
| R,S | 1 mg/kg p.o. (nach 2 Std.) | >5000 mg/kg p.o. |
| | 10 mg/kg p.o. (nach 2 Std.) | |
| | 30 mg/kg p.o. (nach 2 Std.) | |
| | 50 mg/kg p.o. (nach 2 Std.) | |
| R | 30 mg/kg p.o. (nach 2 Std.) | >5000 mg/kg p.o. |
| | 50 mg/kg p.o. (nach 2 Std.) | |
| | 100 mg/kg p.o. (nach 2 Std.) | |
| S | 30 mg/kg p.o. (nach 2 Std.) | >4000 mg/kg p.o. |
| | 50 mg/kg p.o. (nach 2 Std.) | |

Die Verbindung der Formel I kann als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Wie eingangs erwähnt, sind Arzneimittel, enthaltend die Verbindung der Formel I, ebenfalls Gegenstand der vorliegenden Erfindung, weiterhin auch ein Verfahren zur Herstellung derartiger Arzneimittel, welches dadurch gekennzeichnet ist, dass man die Verbindung der Formel I und erwünschtenfalls einen oder mehrere andere therapeutisch wertvolle Stoffe in eine galenische Darreichungsform bringt.

Zur Herstellung von Tabletten, Lacktabletten, Dragées und Hartgelatinekapseln kann die Verbindung der Formel I mit pharmazeutisch inerten, anorganischen oder organischen Excipientien verarbeitet werden. Als solche Excipientien kann man z. B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z. B. vegetabile Oele, Wachse, Fette, halbfeste und flüssige Polyole etc.

Zur Herstellung von Lösungen und Sirupen eignen sich als Excipientien z. B. Wasser, Polyol, Saccharose, Invertzucker, Glukose und dergleichen.

Für Injektionslösungen eignen sich als Excipientien z. B. Wasser, Alkohole, Polyole, Glyzerin, vegetabile Oele etc.

Für Suppositorien eignen sich als Excipientien z. B: natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutische wertvolle Stoffe enthalten.

Erfindungsgemäss kann man die Verbindung der Formel I bei der Bekämpfung bzw. Verhütung der cerebralen Insuffizienz bzw. bei der Verbesserung der intellektuellen Leistungsfähigkeit verwenden, beispielsweise bei cerebralen Insulten, in der Geriatrie, bei Alkoholismus usw. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2 500 mg der Verbindung der Formel I angemessen sein, wobei aber die soeben angegebene obere Grenze auch überschritten werden kann, wenn sich dies als angezeigt erweisen sollte.

In den nachfolgenden Beispielen, welche die vorliegende Erfindung illustrieren, deren Umfang jedoch in keiner Weise einschränken sollen, sind sämtliche Temperaturen in Celsiusgraden angegeben.

### Beispiel 1

a) Zu 4,3 g (R,S)-4-Amino-2-hydroxybuttersäure in 80 ml ionenfreiem Wasser werden unter gutem Rühren 20,0 g 3-Benzyloxy-4-methoxybenzoylchlorid und 20 ml Tetrahydrofuran zugegeben. Danach wird das Gemisch mit 2N Natronlauge auf ein pH von 10,5 gebracht und während 180 Minuten durch Zugabe von 2N Natronlauge bei diesem pH gehalten. Die Suspension wird dann filtriert, worauf man das Filtrat mit Eis versetzt und sein pH mit 25 %iger Salzsäure auf 1 bringt. Der ausgefallene Festkörper wird abfiltriert,

mit Wasser gewaschen, getrocknet und gemahlen, worauf man 800 ml Methylenchlorid zugibt und 1 Stunde zum Rückfluss erhitzt. Der unlösliche Anteil, (R,S)-4-[(3-Benzyloxy-4-methoxybenzoyl)-amino]-2-hydroxybuttersäure, wird abfiltriert und mit Methylenchlorid gewaschen. Durch Einengen des Filtrats kann weitere (R,S)-4-[(3-Benzyloxy-4-methoxybenzoyl)amino]-2-hydroxybuttersäure gewonnen werden ; Smp. 140-141°.

b) 4,0 g (R,S)-4-[(3-Benzyloxy-4-methoxybenzoyl)amino]-2-hydroxybuttersäure und 0,55 g Natrium-Trifluoracetat werden in 24 ml Trifluoressigsäureanhydrid 48 Stunden unter Rühren am Rückfluss gekocht. Nach Eindampfen des Reaktionsgemisches wird der Rückstand viermal mit Toluol geschüttelt, und das Toluol wird danach im Vakuum abgedampft. Der Rückstand, enthaltend (R,S)-1-(3-Benzyloxy-4-methoxybenzoyl)-2-oxo-3-pyrrolidinyl-trifluoracetat, wird in absolutem Methanol 30 Minuten am Rückfluss gekocht. Nach Abdampfen des Methanols wird der Rückstand mit Essigsäureäthylester und Wasser versetzt. Man filtriert den unlöslichen Festkörper ab und erhält (R,S)-1-(3-Benzyloxy-4-methoxybenzoyl)-3-hydroxy-2-pyrrolidinon vom Schmelzpunkt 182-183°. Aus der organischen Phase des Filtrats kann eine weitere Portion dieses Produkts mit demselben Schmelzpunkt isoliert werden.

c) 1,8 g (R,S)-1-(3-Benzyloxy-4-methoxybenzoyl)-3-hydroxy-2-pyrrolidinon werden in 70 ml Tetrahydrofuran gelöst und über 1,5 g 5 %iger Palladium/Kohle mit Wasserstoff bei Atmosphärendruck hydriert. Nach Abfiltrieren des Katalysators und Einengen des Filtrats wird der Rückstand in Diäthyläther bei Raumtemperatur verrührt. Man filtriert den Festkörper ab und erhält so (R,S)-1-(3-Hydroxy-4-methoxybenzoyl)-3-hydroxy-2-pyrrolidinon vom Schmelzpunkt 131-132°.

## Beispiel 2

a) 4,6 g (R,S)-4-[(3-Benzyloxy-4-methoxybenzoyl)amino]-2-hydroxybuttersäure werden in 12 ml Essigsäureanhydrid 15 Minuten am Rückfluss gekocht. Nach Eindampfen des Reaktionsgemisches wird der Rückstand sechsmal mit Toluol geschüttelt, und das Toluol wird danach im Vakuum abgedampft. Man erhält (R,S)-1-(3-Benzyloxy-4-methoxybenzoyl)-2-oxo-3-pyrrolidinylacetat vom Schmelzpunkt 140-141°.

b) 4,60 g (R,S)-1-(3-Benzyloxy-4-methoxybenzoyl)-2-oxo-3-pyrrolidinylacetat werden in 100 ml Essigsäure über 2,0 g 5 %iger Palladiumkohle mit Wasserstoff bei Atmosphärendruck hydriert. Nach Abfiltrieren des Katalysators und Abdampfen der Essigsäure im Vakuum erhält man nach Verrühren in Diäthyläther (R,S)-1-(3-Hydroxy-4-methoxybenzoyl)-2-oxo-3-pyrrolidinylacetat vom Schmelzpunkt 141-142°.

c) Zu 0,50 g gemahlenem (R,S)-1-(3-Hydroxy-4-methoxybenzoyl)-2-oxo-3-pyrrolidinylacetat in 20 ml 0,05 molarem Kalium-Natriumphosphatpuffer vom pH 6,61 werden 1710 Einheiten Esteraseenzym zugegeben, worauf man 195 Minuten bei Raumtemperatur rührt und dann die unlöslichen Anteile abfiltriert. Das Filtrat wird weitere 135 Minuten bei Raumtemperatur gerührt und danach mit Essigester extrahiert. Die Essigesterphase wird mit Wasser gewaschen. Die Wasserphasen werden mit Essigester nachextrahiert. Die vereinigten Essigesterextrakte werden über Natriumsulfat getrocknet, filtriert und eingedampft. Im Rückstand kann durch zweidimensionale Dünnschichtchromatographie (R,S)-1-(3-Hydroxy-4-methoxybenzoyl)-3-hydroxy-2-pyrrolidinon nachgewiesen werden.

## Beispiel 3

a) 6,0 g (R,S)-3-Hydroxy-2-pyrrolidinon werden in 120 ml Pyridin gelöst, worauf man bei 0 bis 5° 24 ml Chlorameisensäurebenzylester zugibt und danach 22 Stunden bei Raumtemperatur rührt. Das Reaktionsgemisch wird eingedampft, worauf man den Rückstand in Toluol verrührt und wiederum eindampft. Der Rückstand wird zwischen Essigsäureäthylester und Wasser verteilt. Die organische Phase wird mit Wasser gewaschen, und die Wasserphasen werden mit Essigsäureäthylester nachextrahiert. Die vereinigten Essigsäureäthylesterphasen werden über Natriumsulfat getrocknet und eingedampft. Der kristalline Rückstand wird in 700 ml Dioxan bei Rückflusstemperatur gelöst und bei + 5° stehen gelassen ; man erhält (R,S)-3-(Benzyloxycarbonyloxy)-2-pyrrolidinon vom Schmelzpunkt 81-82°.

b) 5,0 g (R,S)-3-(Benzyloxycarbonyloxy)-2-pyrrolidinon werden in Tetrahydrofuran mittels Trimethylchlorsilan und Triäthylamin silyliert. Man erhält (R,S)-3-(Benzyloxycarbonyloxy)-1-trimethylsilyl-2-pyrrolidinon vom Schmelzpunkt 56-58°.

c) 3,30 g (R,S)-3-(Benzyloxycarbonyloxy)-1-trimethylsilyl-2-pyrrolidinon werden mit 2,97 g 3-Benzyloxy-4-methoxybenzoylchlorid vermischt, worauf man bei Raumtemperatur rührt. Hierauf wird in einem Oelbad von 100° das entstehende Trimethyldichlorsilan unter vermindertem Druck abdestilliert. Der Rückstand wird zwischen Essigsäureäthylester und Wasser verteilt. Die organische Phase wird mit Aktivkohle behandelt, über Natriumsulfat getrocknet und eingedampft. Nach Verrühren des Rückstandes in Diäthyläther erhält man (R,S)-3-(Benzyloxycarbonyloxy)-1-(3-benzyloxy-4-methoxybenzoyl)-2-pyrrolidinon vom Schmelzpunkt 125-126°.

d) 3,0 g (R,S)-3-(Benzyloxycarbonyloxy)-1-(3-benzyloxy-4-methoxybenzoyl)-2-pyrrolidinon werden in 60 ml Tetrahydrofuran über 1,5 g 5 % Palladiumkohle mit Wasserstoff bei Atmosphärendruck hydriert. Nach Abfiltrieren des Katalysators und Einengen des Filtrats erhält man (R,S)-1-(3-Hydroxy-4-methoxybenzoyl)-3-hydroxy-2-pyrrolidinon, welches nach Verrühren in Diäthyläther bei 125-126° schmilzt.

Beispiel 4

a) Zu 4,3 g (R)-4-Amino-2-hydroxybuttersäure in 80 ml ionenfreiem Wasser werden unter gutem Rühren 20,0 g 3-Benzyloxy-4-methoxybenzoylchlorid und 20 ml Tetrahydrofuran zugegeben. Danach wird das Gemisch mit 2N Natronlauge auf ein pH von 10,5 gebracht und während 200 Minuten durch Zugabe von 2N Natronlauge bei diesem pH gehalten. Die Suspension wird dann filtriert, worauf man das Filtrat mit Eis versetzt und sein pH mit 25 %iger Salzsäure auf 1,4 bringt. Der ausgefallene Festkörper wird abfiltriert, mit Wasser ionenfrei gewaschen, getrocknet und an 90 g Kieselgel (Korngrösse 0,2 bis 0,5 mm) chromatographiert. Die mit Essigsäureäthylester eluierte fast reine (R)-4-[(3-Benzyloxy-4-methoxybenzoyl)amino]-2-hydroxy-buttersäure zeigt nach Umkristallisation aus Acetonitril einen Schmelzpunkt von 138-140°.

b) 5,0 g (R)-4-[(3-Benzyloxy-4-methoxybenzoyl)amino]-2-hydroxy-buttersäure und 0,70 g Natrium-Trifluoracetat werden in 30 ml Trifluoressigsäureanhydrid 48 Stunden unter Rühren am Rückfluss gekocht. Nach Eindampfen des Reaktionsgemisches wird der Rückstand dreimal mit Toluol geschüttelt, und das Toluol wird danach im Vakuum abgedampft. Der Rückstand, enthaltend (R)-1-(3-Benzyloxy-4-methoxybenzoyl)-2-oxo-3-pyrrolidinyltrifluoracetat, wird in absolutem Methanol 30 Minuten am Rückfluss gekocht. Nach Abdampfen des Methanols wird der Rückstand mit Essigsäureäthylester versetzt. Die unlöslichen Anteile werden abfiltriert, und man erhält nach Umkristallisation aus Essigsäureäthylester/n-Hexan (R)-1-(3-Benzyloxy-4-methoxybenzoyl)-3-hydroxy-2-pyrrolidinon vom Schmelzpunkt 164-166° ; $[\alpha]_D^{20}$ : + 143°, $[\alpha]_{546}^{20}$ : + 177°, $[\alpha]_{365}^{20}$ : + 866° (Chloroform, c = 1,0).

c) 2,20 g (R)-1-(3-Benzyloxy-4-methoxybenzoyl)-3-hydroxy-2-pyrrolidinon werden in 60 ml Tetrahydrofuran über 1,80 g 5 %iger Palladiumkohle mit Wasserstoff bei Atmosphärendruck hydriert. Nach Abfiltrieren des Katalysators, Einengen des Filtrates und Umkristallisation des Rückstandes aus Essigsäureäthylester/n-Hexan erhält man (R)-1-(3-Hydroxy-4-methoxybenzoyl)-3-hydroxy-2-pyrrolidinon vom Schmelzpunkt 129-131° ; $[\alpha]_D^{20}$ : + 175°, $[\alpha]_{546}^{20}$ : + 216°, $[\alpha]_{436}^{20}$ : + 451° (Chloroform, c = 1,0).

Beispiel 5

a) Zu 5,06 g (S)-4-Amino-2-hydroxybuttersäure in 130 ml ionenfreiem Wasser werden unter gutem Rühren 15,5 g 3-Benzyloxy-4-methoxybenzoylchlorid und 20 ml Tetrahydrofuran zugegeben. Danach wird das Gemisch mit 2N Natronlauge auf ein pH von 10,5 gebracht und während 180 Minuten durch Zugabe von 2N Natronlauge bei diesem pH gehalten. Die Suspension wird dann filtriert, worauf man das Filtrat mit Eis versetzt, sein pH mit 25 %iger Salzsäure auf 1,4 bringt und mit Essigsäureäthylester extrahiert. Der Eindampfrückstand des Extrakts wird in 240 ml Methylenchlorid am Rückfluss gekocht. Die unlösliche (S)-4-[(3-Benzyloxy-4-methoxybenzoyl)amino]-2-hydroxy-buttersäure wird abfiltriert ; sie schmilzt nach Umkristallisation aus Acetonitril bei 138-140°.

b) 6,5 g (S)-4-[(3-Benzyloxy-4-methoxybenzoyl)amino]-2-hydroxybuttersäure und 1,0 g Natrium-Trifluoracetat werden in 40 ml Trifluoressigsäureanhydrid 48 Stunden unter Rühren am Rückfluss gekocht. Nach Eindampfen des Reaktionsgemisches wird der Rückstand dreimal mit Toluol geschüttelt, und das Toluol wird danach im Vakuum abgedampft. Der Rückstand, enthaltend (S)-1-(3-Benzyloxy-4-methoxybenzoyl)-2-oxo-3-pyrrolidinyltrifluoracetat wird in 40 ml absolutem Methanol 30 Minuten am Rückfluss gekocht. Danach wird bei Raumtemperatur 1 Stunde nachgerührt, worauf der Festkörper abfiltriert und mit Methanol gewaschen wird. Man erhält (S)-1-(3-Benzyloxy-4-methoxybenzoyl)-3-hydroxy-2-pyrrolidinon, welches nach Umkristallisieren aus Methanol bei 166-167° schmilzt ; $[\alpha]_D^{20}$ : − 146°, $[\alpha]_{546}^{20}$ : − 180°, $[\alpha]_{365}^{20}$ : − 879° (Chloroform, c = 1,0).

c) 2,40 g (S)-1-(3-Benzyloxy-4-methoxybenzoyl)-3-hydroxy-2-pyrrolidinon werden in 100 ml Tetrahydrofuran über 2,00 g 5 %iger Palladiumkohle mit Wasserstoff bei Atmosphärendruck hydriert. Nach Abfiltrieren des Katalysators, Einengen des Filtrates und Umkristallisation aus Essigsäureäthylester-Diäthyläther erhält man (S)-1-(3-Hydroxy-4-methoxybenzoyl)-3-hydroxy-2-pyrrolidinon vom Schmelzpunkt 131-132° ; $[\alpha]_D^{20}$ : − 180°, $[\alpha]_{546}^{20}$ : − 223°, $[\alpha]_{436}^{20}$ : − 464°, (Chloroform, c = 1,0).

Beispiel A

1-(3-Hydroxy-4-methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 25 mg |
| Milchzucker (pulverisiert) | 180 mg |
| Maisstärke (weiss) | 275 mg |
| Polyvinylpyrrolidon | 15 mg |
| Magnesiumstearat | 5 mg |
| | 500 mg |

Der feingemahlene Wirkstoff, der pulverisierte Milchzucker und ein Teil der weissen Maisstärke

werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Grösse verpresst.

Beispiel B

1-(3-Hydroxy-4-methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 20 mg |
| Maisstärke (weiss) | 220 mg |
| Milchzucker | 70 mg |
| Mikrokristalline Cellulose | 40 mg |
| Polyvinylpyrrolidon | 20 mg |
| Natrium-carboxymethylstärke | 23 mg |
| Magnesiumstearat | 2 mg |
| | 395 mg |

Der feingemahlene Wirkstoff, ein Teil der weissen Maisstärke, der Milchzucker, die mikrokristalline Cellulose und das Polyvinylpyrrolidon werden miteinander vermischt. Die Mischung wird gesiebt und mit dem Rest der weissen Maisstärke und Wasser zu einem Granulat verarbeitet, welches getrocknet und gesiebt wird. Dann gibt man die Natrium-carboxymethylstärke und das Magnesiumstearat zu, vermischt und verpresst das Gemisch zu Tabletten geeigneter Grösse, welche eine Kreuzbruchrille aufweisen.

Beispiel C

1-(3-Hydroxy-4-methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 125 mg |
| Maisstärke (weiss) | 560 mg |
| Milchzucker | 165 mg |
| Mikrokristalline Cellulose | 70 mg |
| Polyvinylpyrrolidon | 35 mg |
| Natrium-carboxymethylstärke | 40 mg |
| Magnesiumstearat | 5 mg |
| | 1 000 mg |

Der feingemahlene Wirkstoff, ein Teil der weissen Maisstärke, der Milchzucker, die mikrokristalline Cellulose und das Polyvinylpyrrolidon werden miteinander vermischt. Die Mischung wird gesiebt und mit dem Rest der weissen Maisstärke und Wasser zu einem Granulat verarbeitet, welches getrocknet und gesiebt wird. Dann gibt man die Natrium-carboxymethylstärke und das Magnesiumstearat zu, vermischt und verpresst das Gemisch zu Tabletten geeigneter Grösse, welche eine Kreuzbruchrille aufweisen.

Beispiel D

1-(3-Hydroxy-4-methoxybenzoyl)-3-hydroxy-2-pyrrolidinon wird als Wirkstoff zur Herstellung von Doppelampullen folgender Zusammensetzung verwendet :

| | |
|---|---|
| Wirkstofflösung | |
| Wirkstoff | 25 mg |
| Polyäthylenglykol | ad 5 mg |
| | |
| Diluens | |
| Wasser zu Injektionszwecken | 5 ml |

Vor der Injektion ist das Diluens dem Inhalt der Wirkstoffampulle zuzufügen. Man erhält 10 ml einer spritzfertigen Lösung, enthaltend 25 mg Wirkstoff.

Beispiel E

1-(3-Hydroxy-4-methoxybenzoyl)-3-hydroxy-2-pyrrolidinon wird als Wirkstoff zur Herstellung von

Doppelampullen folgender Zusammensetzung verwendet :

| | |
|---|---|
| Wirkstofflösung | |
| Wirkstoff | 25 mg |
| Glycofurol | ad 3,5 ml |
| Diluens | |
| Natriumchlorid | 67,5 mg |
| Wasser zu Injektionszwecken | ad 6,5 ml |

Vor der Injektion ist das Diluens dem Inhalt der Wirkstoffampulle zuzufügen. Man erhält 10 ml einer spritzfertigen Lösung, enthaltend 25 mg Wirkstoff.

Beispiel F

1-(3-Hydroxy-4-methoxybenzoyl)-3-hydroxy-2-pyrrolidinon wird als Wirkstoff zur Herstellung von Doppelampullen folgender Zusammensetzung verwendet :

| | |
|---|---|
| Wirkstofflösung | |
| Wirkstoff | 25 mg |
| Polyäthylenglykol | 1,5 ml |
| Glycofurol | ad 4 ml |
| Diluens | |
| Wasser zu Injektionszwecken | 6 ml |

Vor der Injektion ist das Diluens dem Inhalt der Wirkstoffampulle zuzufügen. Man erhält 10 ml einer spritzfertigen Lösung, enthaltend 25 mg Wirkstoff.

**Patentansprüche** (für die Vertragsstaaten : BE, CH, DE, FR, IT, LI, LU, NL, SE)

1. 1-(3-Hydroxy-4-methoxybenzoyl)-3-hydroxy-2-pyrrolidinon der Formel

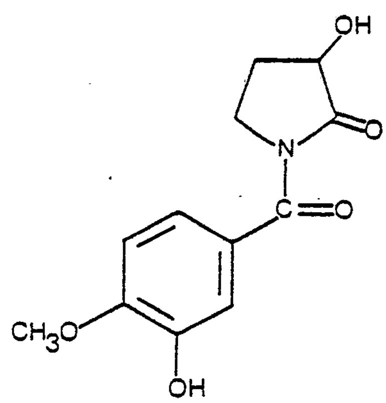

(I)

2. (R,S)-1-(3-Hydroxy-4-methoxybenzoyl)-3-hydroxy-2-pyrrolidinon.
3. (R)-1-(3-Hydroxy-4-methoxybenzoyl)-3-hydroxy-2-pyrrolidinon.
4. (S)-1-(3-Hydroxy-4-methoxybenzoyl)-3-hydroxy-2-pyrrolidinon.
5. Verbindungen der allgemeinen Formel

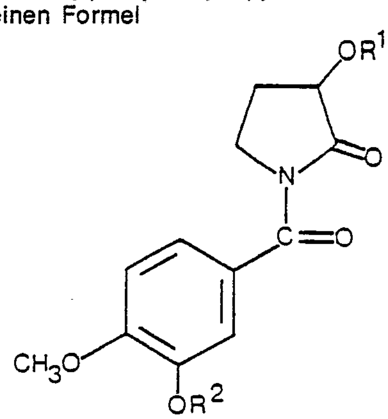

(II)

worin eines von $R^1$ und $R^2$ eine Schutzgruppe und das andere Wasserstoff oder eine Schutzgruppe bedeuten.

6. Verbindung gemäss einem der Ansprüche 1 bis 4 als pharmazeutischer Wirkstoff.

7. Verbindung gemäss einem der Ansprüche 1 bis 4 als der cerebralen Insuffizienz entgegenwirkender bzw. die intellektuelle Leistungsfähigkeit verbessernder Wirkstoff.

8. Verfahren zur Herstellung der Verbindung gemäss einem der Ansprüche 1 bis 4, dadurch gekennzeichnet, dass man aus einem Pyrrolidin-Derivat der allgemeinen Formel

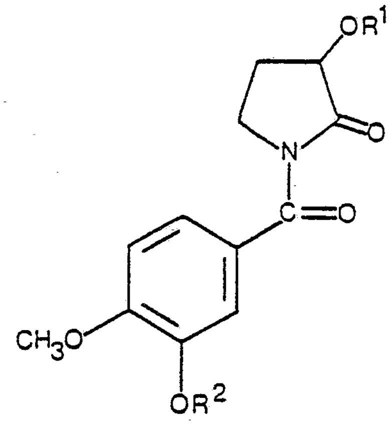

(II)

worin eines von $R^1$ und $R^2$ eine Schutzgruppe und das andere Wasserstoff oder eine Schutzgruppe bedeuten, die Schutzgruppe(n) entfernt.

9. Arzneimittel, enthaltend die Verbindung gemäss einem der Ansprüche 1 bis 4.

10. Der cerebralen Insuffizienz entgegenwirkendes bzw. die intellektuelle Leistungsfähigkeit verbesserndes Mittel, enthaltend die Verbindung gemäss einem der Ansprüche 1 bis 4.

**Patentansprüche** (für den Vertragsstaat AT)

1. Verfahren zur Herstellung von 1-(3-Hydroxy-4-methoxybenzoyl)-3-hydroxy-2-pyrrolidinon der Formel

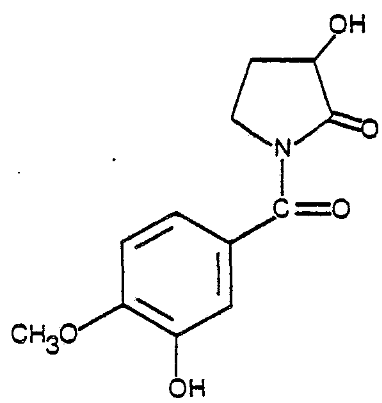

(I)

dadurch gekennzeichnet, dass man aus einem Pyrrolidin-Derivat der allgemeinen Formel

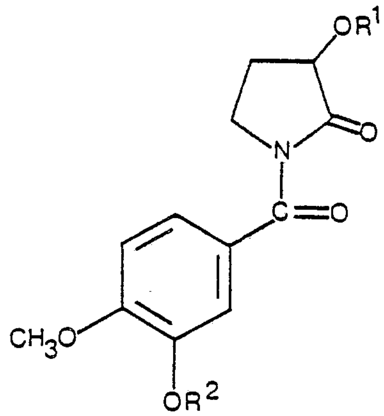

(II)

worin eines von $R^1$ und $R^2$ eine Schutzgruppe und das andere Wasserstoff oder eine Schutzgruppe bedeuten, die Schutzgruppe(n) entfernt.

2. Verfahren nach Anspruch 1, dadurch gekennzeichnet, dass man (R,S)-1-(3-Hydroxy-4-methoxybenzoyl)-3-hydroxy-2-pyrrolidinon herstellt.

3. Verfahren nach Anspruch 1, dadurch gekennzeichnet, dass man (R)-1-(3-Hydroxy-4-methoxybenzoyl)-3-hydroxy-2-pyrrolidinon herstellt.

4. Verfahren nach Anspruch 1, dadurch gekennzeichnet, dass man (S)-1-(3-Hydroxy-4-methoxybenzoyl)-3-hydroxy-2-pyrrolidinon herstellt.

**Claims** (for the Contracting States : BE, CH, DE, FR, IT, LI, LU, NL, SE)

1. 1-(3-Hydroxy-4-methoxybenzoyl)-3-hydroxy-2-pyrrolidinone of the formula

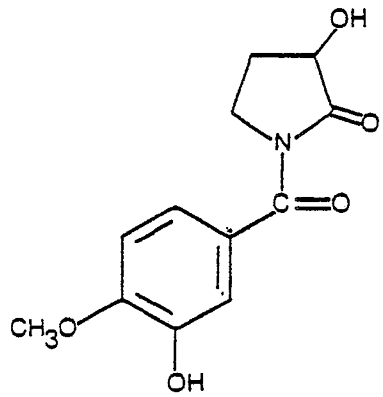

(I)

2. (R,S)-1-(3-Hydroxy-4-methoxybenzoyl)-3-hydroxy-2-pyrrolidinone.
3. (R)-1-(3-Hydroxy-4-methoxybenzoyl)-3-hydroxy-2-pyrrolidinone.
4. (S)-1-(3-Hydroxy-4-methoxybenzoyl)-3-hydroxy-2-pyrrolidinone.
5. Compounds of the general formula

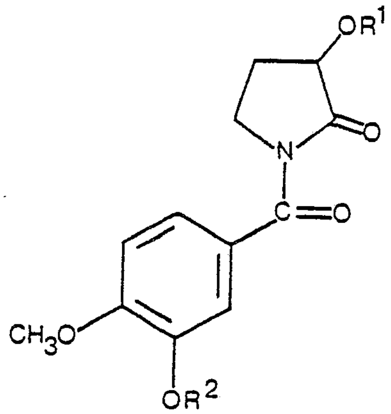

(II)

wherein one of $R^1$ and $R^2$ signifies a protecting group and the other signifies hydrogen or a protecting group.

6. The compound in accordance with any one of claims 1 to 4 as a pharmaceutically active substance.

7. The compound in accordance with any one of claims 1 to 4 as an active substance counteracting cerebral insufficiency or improving intellectual capacity.

8. A process for the manufacture of the compound in accordance with any one of claims 1 to 4, characterized by removing the protecting group(s) from a pyrrolidine derivative of the general formula

(See Figure page 14)

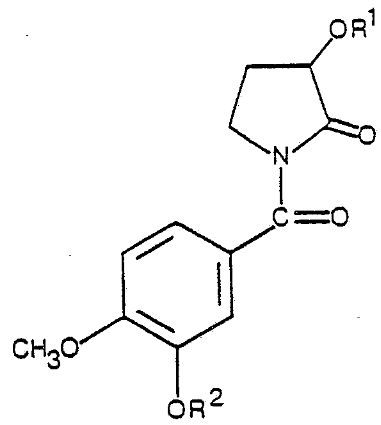

(II)

wherein one of $R^1$ and $R^2$ signifies a protecting group and the other signifies hydrogen or a protecting group.

9. A medicament containing the compound in accordance with any one of claims 1 to 4.

10. A medicament counteracting cerebral insufficiency or improving intellectual capacity, containing the compound in accordance with any one of claims 1 to 4.

**Claims** (for the Contracting State AT)

1. A process for the manufacture of 1-(3-hydroxy-4-methoxybenzoyl)-3-hydroxy-2-pyrrolidinone of the formula

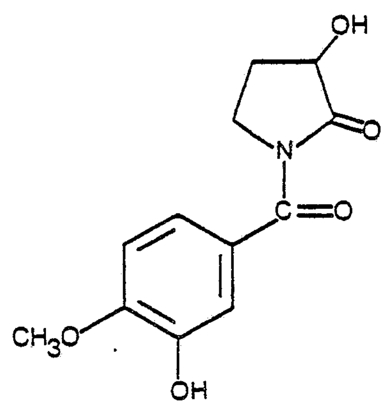

(I)

characterized by removing the protecting group(s) from a pyrrolidine derivative of the general formula

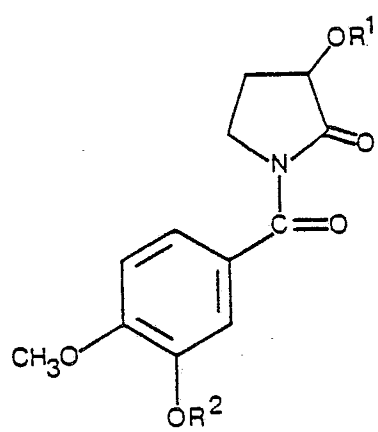

(II)

wherein one of $R^1$ and $R^2$ signifies a protecting group and the other signifies hydrogen or a protecting group.

2. A process according to claim 1, characterized in that (R,S)-1-(3-hydroxy-4-methoxybenzoyl)-3-hydroxy-2-pyrrolidinone is manufactured.

3. A process according to claim 1, characterized in that (R)-1-(3-hydroxy-4-methoxybenzoyl)-3-hydroxy-2-pyrrolidinone is manufactured.

4. A process according to claim 1, characterized in that (S)-1-(3-hydroxy-4-methoxybenzoyl)-3-hydroxy-2-pyrrolidinone is manufactured.

**Revendications** (pour les Etats contractants : BE, CH, DE, FR, IT, LI, LU, NL, SE)

1. La 1-(3-hydroxy-4-méthoxybenzoyl)-3-hydroxy-2-pyrrolidinone de formule

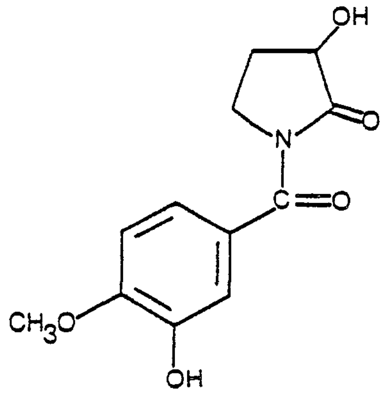

(I)

2. La (R,S)-1-(3-hydroxy-4-méthoxybenzoyl)-3-hydroxy-2-pyrrolidinone.
3. La (R)-1-(3-hydroxy-4-méthoxybenzoyl)-3-hydroxy-2-pyrrolidinone.
4. La (S)-1-(3-hydroxy-4-méthoxybenzoyl)-3-hydroxy-2-pyrrolidinone.
5. Composés de formule générale

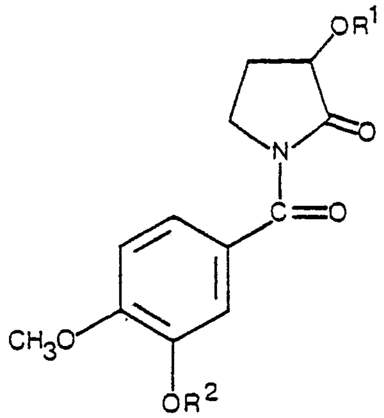

(II)

dans laquelle l'un des symboles $R^1$ et $R^2$ représente un groupe protecteur et l'autre l'hydrogène ou un groupe protecteur.

6. Composé selon l'une des revendications 1 à 4, en tant que substance active pharmaceutique.

7. Composé selon l'une des revendications 1 à 4, en tant que substance active agissant contre l'insuffisance cérébrale ou améliorant la capacité intellectuelle.

8. Procédé de préparation du composé selon l'une des revendications 1 à 4, caractérisé en ce que, dans un dérivé de la pyrrolidine de formule générale

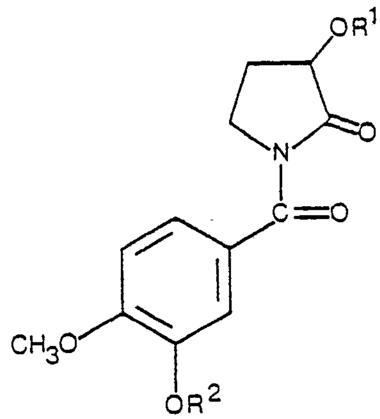

(II)

dans laquelle l'un des symboles $R^1$ et $R^2$ représente un groupe protecteur et l'autre l'hydrogène ou un groupe protecteur, on élimine le ou les groupes protecteurs.

9. Médicaments contenant le composé selon l'une des revendications 1 à 4.

10. Agent agissant contre l'insuffisance cérébrale ou améliorant la capacité intellectuelle, contenant le composé selon l'une des revendications 1 à 4.

**Revendications** (pour l'Etat contractant AT)

1. Procédé de préparation de la 1-(3-hydroxy-4-méthoxybenzoyl)-3-hydroxy-2-pyrrolidinone de formule

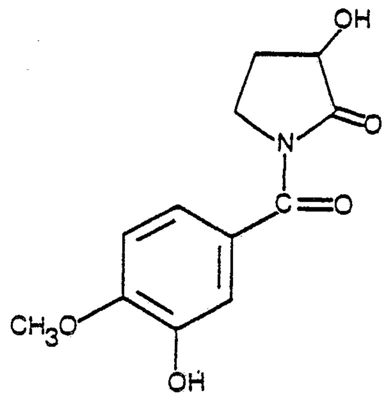

(I)

caractérisé en ce que, dans un dérivé de la pyrrolidine répondant à la formule générale

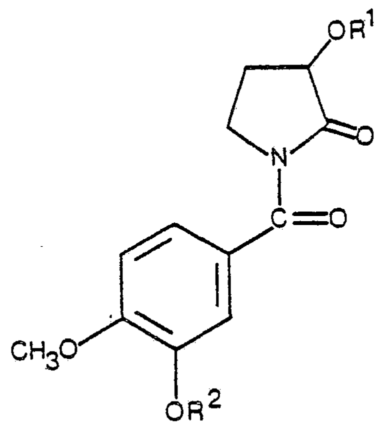

(II)

dans laquelle l'un des symboles $R^1$ et $R^2$ représente un groupe protecteur et l'autre l'hydrogène ou un groupe protecteur, on élimine le ou les groupes protecteurs.

2. Procédé selon la revendication 1, caractérisé en ce que l'on prépare la (R,S)-1-(3-hydroxy-4-méthoxybenzoyl)-3-hydroxy-2-pyrrolidinone.

3. Procédé selon la revendication 1, caractérisé en ce que l'on prépare la (R)-1-(3-hydroxy-4-méthoxybenzoyl)-3-hydroxy-2-pyrrolidinone.

4. Procédé selon la revendication 1, caractérisé en ce que l'on prépare la (S)-1-(3-hydroxy-4-méthoxybenzoyl)-3-hydroxy-2-pyrrolidinone.